(19) Europäisches Patentamt
European Patent Office
Office européen des brevets

(11) Publication number: 0 502 333 A1

# (12) EUROPEAN PATENT APPLICATION

(21) Application number: **92102060.8**

(22) Date of filing: **07.02.92**

(51) Int. Cl.5: **C07B 39/00**, C07C 17/10, C07C 25/18, C07C 43/29, C08K 5/03

(30) Priority: **25.02.91 US 660810**

(43) Date of publication of application: **09.09.92 Bulletin 92/37**

(84) Designated Contracting States: **BE DE ES FR GB IT NL**

(71) Applicant: **ETHYL CORPORATION**
**451 Florida Boulevard**
**Baton Rouge, Louisiana 70801(US)**

(72) Inventor: **Brackenridge, David Ross**
**5232 Halls Ferry**
**Baton Rouge, Louisiana 70817(US)**
Inventor: **Murray, William Thomas**
**10795 Mead Road, Apt. 1302**
**Baton Rouge, Louisiana 70816(US)**
Inventor: **Davis, Robert Lynn**
**1343 Dahlia**
**Baton Rouge, Louisiana 70808(US)**
Inventor: **Ramezanian, Merrikh Sabahi**
**851 Shadow Oak Drive**
**Baton Rouge, Louisiana 70810(US)**

(74) Representative: **Sandmair, Kurt, Dr. Dr.**
**Patentanwälte Schwabe, Sandmair, Marx**
**Stuntzstrasse 16**
**W-8000 München 80(DE)**

## (54) Bromination process.

(57) A process is disclosed for preparing a mixture of brominated, non-condensed ring polyaromatics, which process comprises brominating the precursor non-condensed ring polyaromatic in the presence of a bromination catalyst. The mixture has an average bromine number of 5.8 to 6.2, more than about 55 GC area percent of the hexabromo homolog, and a reduced amount of light-end impurities.

EP 0 502 333 A1

This invention relates to a flame retardant product comprising a novel mixture of halogenated polyaromatic compounds having a narrow melting point range and the process therefor.

Mixtures of brominated non-condensed ring polyaromatics are known. For example, brominated diphenyl oxide mixtures having an average bromine number of from 7.0 to 7.7 are sold commercially as flame retardants for use in thermoplastic formulations. These mixtures conventionally contain 0-2 weight percent hexabromodiphenyl oxide, 40-55 weight percent heptabromodiphenyl oxide, 30-40 weight percent octabromodiphenyl oxide, 5-15 weight percent nonabromodiphenyl oxide and 0-2 weight percent decabromodiphenyl oxide. Other brominated non-condensed ring polyaromatic mixtures are disclosed in: U.S. 3,833,674; U.S 4,717,776; and U.S. 4,740,629.

The particular distribution of the various bromo homologs in the mixture will determine the mixture's average bromine number and its possible effect on the physical properties of articles made with thermoplastic formulations containing such mixtures. It is generally desirable to have a high average bromine number since the amount of bromine in the mixture is directly tied to the flame retardant effect per unit weight of the mixture in the formulation. Obtainment of high average bromine number has heretofore been accomplished by producing mixtures which contain large amounts of the hepta- and octabromo homologs. While these higher average bromine numbers are beneficial in regard to minimizing the amount of mixture needed to obtain a certain flame retardancy level, the use of large amounts of hepta- and octabromo homologs is not without a significant penalty. It has been found that such homologs contribute to a reduction in an articles' impact strength, which reduction is similar to that which occurs when a filler, e.g., talc, $Mg(OH)_2$, or ZnO, is present in the article. To give the mixture a less filler-type nature, the mixture should contain more of the less brominated homologs, e.g., the penta- and hexabromo homologs, and less of the hepta- and octabromo homologs. The lower brominated homologs, i.e., hexabromo and below, will give the mixture a plasticizer-type component which can be balanced against the filler-type component provided by the higher bromo homologs. A major problem with this approach is that the art has not developed a process which produces a mixture which contains significant amounts of the less brominated homologs and which, has, at the same time, a sufficiently high enough average bromine number.

This invention relates to a process for preparing a mixture of brominated non-condensed ring polyaromatics having an average of about 6 bromine atoms per molecule and a reduced amount of light-end impurities, comprising: adding bromine to a reactor initially containing a reaction mass comprising a solvent, a catalytic amount of a bromination catalyst, and a non-condensed ring polyaromatic; maintaining the reaction mass during the bromine addition at a temperature of from 15° to 40° C; terminating the addition of bromine when sufficient bromine has been added to obtain the mixture of polyaromatics having an average of about 6 bromine atoms per molecule of polyaromatic; and after at least substantially all of the bromine has reacted, recovering the brominated non-condensed ring polyaromatic mixture from the reaction mass, the recovered mixture containing more than about 55 %, based on gas chromatographic area percent, of a brominated polyaromatic having about 6 bromine atoms per molecule.

The non-condensed ring polyaromatic reactant used in the process of this invention can be represented by the formula

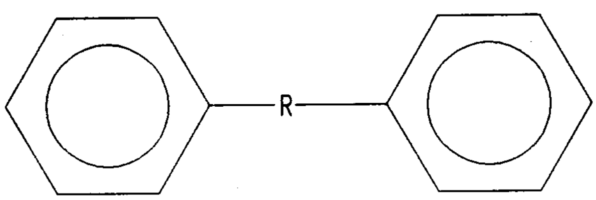

wherein R is an alkylene group containing 1 to 10 carbon atoms, an oxygen atom, a sulfur atom, an oxyalkylene group (-O-R-) of up to 6 carbon atoms, an oxyalkyleneoxy group (-O-R-O-) of up to 6 carbon atoms or a carbon single bond. Preferred R groups are methylene and ethylene which give, respectively, the preferred reactants, diphenylmethane and 1,2-diphenylethane. Exemplary of other polyaromatic reactants are: diphenyl oxide, diphenyl, diphenylsulfide, 1,3-diphenoxyethane, 1-methyl-1,2-diphenyl-ethane, 1,3-diphenylpropane, 1,4-diphenylbutane, 1,6-diphenylhexane, 2,3-dimethyl-1,4-diphenylbutane,2-ethyl-3-methyl-1,4-diphenylbutane,2-methyl-1,6-diphenylhexane, 1,9-diphenylnonane and 1,10-diphenyldecane.

Based upon present day and anticipated market demand for certain mixtures of this invention, diphenyl oxide and 1,2-diphenylethane are the preferred reactants. The diphenyl oxide has been in commercial use for several years and is commercially available. Diphenylalkanes can be produced by various routes. For example, CA 97 38651d (Japanese Kokai 82/45114) and CA 46 7084g disclose the reaction of benzene and

ethylene dihalide in the presence of aluminum trichloride to yield diphenylethane. Another process for producing diphenylalkane includes the oxidative dimerization of toluene at a temperature of at least 400°C in the presence of a metal oxide catalyst to yield diphenylethane and diphenylalkene. The latter product is then hydrogenated to remove the olefinic unsaturation.

It is not uncommon for the diphenylalkane reactant to be accompanied by various impurities. Diminishing the impurity content can be accomplished in a conventional manner, for example, the diphenylalkane can be recrystallized.

The polyaromatic reactant, catalyst and solvent can be provided to the reactor in any order and in any combination. A preferred manner of addition is to first add a solution of the solvent and the polyaromatic reactant to the reactor and then to add the bromination catalyst. It is preferred that the addition of the materials be at a temperature which is at least near the temperature at which the bromine addition will occur, i.e., 10°C to 40°C. This is not to say that addition cannot occur at other temperatures. However, when the addition is at low temperatures, e.g., 0°C-10°C, care must be taken to prevent atmospheric moisture from being aspirated into the reactor when the reactor contents cool to the bromine addition temperature. The presence of water in the reactor is not desirable as water can deactivate the catalyst. Cooling of the reactor contents may occur naturally as the solvent and non-condensed ring polyaromatic are mixed or by the application of a cooling medium to the shell of the reactor.

The solvent used must be one in which the polyaromatic reactant is substantially soluble and in which the produced mixture is substantially insoluble, i.e., at least about 60 weight percent is insoluble. The solvent should also be a liquid during the bromination of the polyaromatic reactant and substantially inert to the process. Generally, halogenated lower alkanes are suitable. Exemplary solvents are methylene bromide, methylene chloride, ethylene dibromide, ethylene dichloride, bromochloroethane, and mixtures thereof. When using ethylene dichloride, process temperatures which promote transhalogenation of the solvent should be avoided. Preferred solvents are methylene bromide, methylene chloride and mixtures thereof. The most preferred solvent is methylene bromide.

The amount of solvent used is that amount which will at least provide a stirrable reaction mass. Generally, from 150 to 800 mL of solvent per mole of polyaromatic reactant is suitable. Preferably, from 180 mL to 500 mL of solvent per mole of polyaromatic reactant is used, and most preferably, from 200 mL to 400 mL of solvent per mole of polyaromatic reactant is used. It has been found that by utilizing the minimum amount of solvent required to obtain an easily stirred reaction mass, recovery of a product having an average of about 6 bromine atoms per molecule is greatly enhanced.

A key feature of this invention is the obtainment of a product with a narrow melting point range and containing a reduced amount of light end impurities. The produce of this invention with a narrow melting point range is particularly suited to recovery by solvent stripping in boiling water. Recovery of product by this procedure is simpler and less costly than other product purification techniques heretofore practiced. Having a reduced amount of light end impurities in the flame retardant product reduces the tendency of the flame retardant to cause bloom in flame retardant formulations.

By reduced amount of light end impurities is meant an amount which is so insignificant that flame retardant formulations containing the product exhibit less bloom than formulations containing prior art products as flame retardants. Preferably, the amount of light end impurities, based on GC area percent, will be less than about 1%, more preferably, less than about 0.5% and most preferably, less than about 0.3%.

The bromination catalyst used in the process of this invention is conventional and is available from commercial sources or can be readily made by those skilled in the art. Bromination catalysts which may be used include iron; aluminum; zirconium; $ZrCl_4$; $FeCl_3$; $FeBr_3$; and a 1:1 ratio of $FeCl_3 \cdot CH_3NO_2$, $FeBr_3 \cdot CH_3NO_2$; or mixtures thereof. The most preferred catalysts are, at least initially, in the form of $FeCl_3$, or $FeBr_3$ or mixtures thereof.

Since the catalytic activity of the catalyst is degraded by contact with water, at least near-anhydrous conditions should be present before and during the reaction of the polyaromatic reactant and bromine. The catalytic quantities used in the process range from 1 weight percent to 10 weight percent of the total weight of the polyaromatic to be brominated. Preferred amounts are within the range of from 2 weight percent to 5 weight percent of the total weight of the polyaromatic reactant.

Prior to the addition of bromine to the reactor, the polyaromatic reactant, solvent and catalyst in the reactor are at a temperature which is below about 40° C, and preferably between 20° C and 30° C. The temperature can be obtained by charging heated polyaromatic reactant, solvent and/or catalyst to the reactor so as to have the reactor contents at the desired bromine addition temperature. Another method is to charge the three components to the reactor at room temperature and then heat the charge to the desired temperature. During the reactor charging, it is prudent, as before noted, to prevent atmospheric moisture from being aspirated into the reactor.

The bromine addition to the contents of the reactor should occur soon after the polyaromatic reactant, solvent and catalyst have been charged. The amount of bromine added is that amount which will give the recovered mixture an average bromine number, based upon GC area percent, which is within the range of from 5.8 to 6.2. Since all of the bromine which is added to the reactor is reacted, and since it takes one mole of bromine, i.e., $Br_2$, per mole of polyaromatic reactant to effect the placement of one bromine atom on the ring, the number of moles of bromine added will substantially equal the average bromine number of the recovered mixture. The term "substantially" is used to describe this equality since it is possible that some of the bromine added will be lost from the reaction due to its entrainment in the stream of HBr being evolved from the reactor contents and/or due to any competing side reactions. Generally, the losses are not great, say 0.50 percent of the bromine added. However, to obtain the desired average bromine number, any losses must be made up by the addition of the excess "make-up" bromine. Thus, in most instances the amount of bromine added will be in slight molar excess of that which equals the average bromine number. For example, to obtain an average bromine number of six, about 6.04 moles of bromine are usually added. In most instances, from 6.0 to 6.04 moles of bromine will be added.

The bromine can be added to the reactor at any rate which does not cause the reaction mass to overheat and/or does not cause an evolution of by-product HBr which is so great that a safety hazard is created. From a process efficiency standpoint, it is preferable that the addition rate be as rapid as is possible without realization of significant overheating and/or safety problems. Generally, for lab scale processes, the bromine addition rate is preferably from 0.5 mL/min to 1.5 mL/min. Determining the optimum addition rates for large scale processes will be dependent upon reactor configuration, reaction mass size, reactor cooling equipment available and process economics. The optimum addition rate is best determined empirically for each different process size and equipment configuration used.

After the bromine addition is complete, the reaction mass is allowed to undergo a ride period until at least substantially all of the bromine has been reacted. An indication that the bromination reaction is complete is the cessation of HBr evolution from the reactor contents. This indication that all of the bromine has reacted is convenient to use with large scale processes.

The ride time is affected by the temperature of the reactor contents after the bromine addition. Shorter ride times are associated with higher reaction mass temperatures and longer ride times are associated with lower temperatures. It is preferred that the temperature of the reactor contents be raised to be within the range of from 40 °C to 60 °C during the ride period. Generally, it is not desirable to let the temperature of the reactor contents go above about 80 °C as adverse side reactions or solvent loss can occur. The maximum temperature is somewhat determined by the particular solvent used. For the preferred solvent, methylene bromide, the maximum temperature during the ride time should not exceed about 60 °C.

The temperature of the reactor contents during the ride time can be raised by applying heat thereto. It is preferred, from an operations standpoint, to allow the temperature of the reactor contents to rise naturally after the bromine addition until the temperature rise rate becomes unacceptably slow. Then heat can be applied to bring the reactor contents to the selected maximum temperature.

After the reaction between the bromine and the polyaromatic reactant has at least substantially ceased, the brominated mixture is recovered from the reaction contents. Some of the mixture substituents may be dissolved in the solvent and they need to be at least partially recovered therefrom. One technique that can be used is to contact the reactor contents with a $C_1$ to $C_4$ alkanol. The alkanol acts as a precipitating agent to precipitate at least a portion of the dissolved mixture substituents from the solvent. A preferred alkanol is methanol. The reactor contents and alkanol are brought into contact by adding one to the other. The amount of alkanol used can be within the rage of from 1 to 3 volumes of alkanol per volume of the reactor contents. There is no real upper limit to the amount of alkanol that can be used; however, secondary considerations, such as reactor size and process economics, will determine the amount which is sensibly used. The lowest amount of alkanol that is used is that amount which is capable of effecting the recovery sought. The temperature at which the reactor contents and alkanol are contacted is not critical and any convenient temperature can be used. Preferred temperatures are within the range of from ambient temperature to 65 °C. The contact can be maintained up to two hours to ensure the highest degree of precipitation of the brominated polyaromatic mixture substituents which were in the solvent. Lesser times can be used since most substituents will precipitate out almost immediately. The resultant solid portion of the reactor contents, which comprises the brominated polyaromatic mixture, is recovered by conventional liquid-solid separation methods, e.g., filtration or centrifugation.

Another technique for recovering the brominated polyaromatic mixture involves flashing the solvent from the reaction mass by contacting the reaction mass with hot water, i.e., water at a temperature sufficient to effect flash vaporization of the solvent from the reaction mass. The residue remaining after flashing the solvent is comprised principally of the brominated polyaromatic mixture. This method has an added

advantage in that the water will deactivate as well as solubilize the catalyst.

To reduce the impurities in the recovered brominated polyaromatic mixture, it can be washed with alkanol, caustic, water or all three. After washing, the mixture is then dried and milled, if desired, to yield a particulate product.

As before noted, the novel brominated polyaromatic mixtures produced by the process of the invention are characterized in that:

(1) they have an average bromine number, based upon GC area percent, within the rage of from 5.8 to 6.2;

(2) the hexabromo homolog in the mixtures is present in an amount greater than any other homolog;

(3) the mixture has a reduced amount of light end impurities; and

(4) the mixture has a narrow melting point range.

Even though the above specifies that the predominant homolog is the hexabromo homolog, other bromo homologs will probably be present. For example, when the polyaromatic reactant is diphenylethane, tetrabromodiphenylethane, pentabromodiphenyl-ethane, heptabromodiphenylethane and octabromodiphenylethane can be present.

The average bromine number is defined as the average number of bromine atoms per molecule of brominated polyaromatic in the mixture. The average bromine number can be calculated by multiplying the gas chromatographic (GC) area percent or the weight percent of each bromo homolog in the mixture by the number of bromine atoms in that homolog, adding the resulting products and dividing the sum by 100. There will be a slight variation between the average bromine number obtained when using the GC area percent and when using weight percent. This variation can exist because the GC area percent does not always accurately reflect the quantitative relationship between the different bromo homologs in the mixture. The inaccuracy is due to the GC response being different for various of the bromo homologs in the mixture. The variation between GC area percent and weight percent can be resolved by multiplying the GC response factor for each bromo homolog times the GC area percent for that homolog. The product will give the weight percent. For the mixtures of this invention, preferred average bromine numbers, based upon GC area percent, are within the range of from 5.6 to 6.4, with an average bromine number of from 5.8 to 6.2 being most preferred. Most highly preferred are average bromine numbers of 6.0 to 6.1.

For the purposes of obtaining the GC area percents and the identities of the bromo homologs which form the mixtures produced by the process of this invention, a combination of gas chromatography and mass spectrometry can be used. The mass spectrometer is used to identify each bromo homolog and correlate its identity with the particular peak(s) and retention time(s) shown by the gas chromatogram.

It is recognized that the GC area percent values for each bromo homolog may vary slightly dependent upon the particular gas chromatograph used and upon the analytical conditions used in operating the gas chromatograph. To provide a standard for obtaining the GC area percent values used herein, the following is given:

| Gas Chromatography | |
|---|---|
| Instrument | Hewlett Packard 5890 |
| Column | 10 meter, DB-1 methyl silicone megabore, made by J&W Scientific of California |
| Program | 200-320 °C, at 5.5 °/min. + 8 min. hold at 320 °C |
| Injector | 285 °C, split injector, Hewlett Packard |
| Detector | 325 °C FID (flame ionization detector) |
| Sample Size | 1 µl (1% soln) + 1 µl methylene bromide plug |

The response factors used in obtaining the weight percent values recited herein are approximates which are based upon empirical observation and experience in quantifying the bromo homologs in various flame retardants.

| Bromo homolog | Response Factor |
|---|---|
| DPO/DPE-$BR_4$ | 0.85 |
| DPO/DPE-$BR_5$ | 0.85 |
| DPO/DPE-$BR_6$ | 0.9 |
| DPO/DPE-$BR_7$ | 0.9 |
| DPO/DPE-$BR_8$ | 1.0 |
| DPO/DPE-$BR_9$ | 1.1 |
| DPO/DPE-$BR_{10}$ | 1.1 |

More exact response factors can be obtained by conventionally comparing the GC area percents of a known amount of each brominated homolog against one another.

On the basis of GC area percent, a typical bromo homolog distribution for brominated diphenylethane mixtures of this invention is, 5-12 GC area percent pentabromodiphenylethane, 75-90 GC area percent hexabromodiphenylethane, 3-10 GC area percent heptabromodiphenylethane, and 0-1 GC area percent octabromodiphenylethane.

When the mixtures are derived from diphenyl oxide a bromo homolog distribution similar to that for diphenylethane can be obtained by use of the process of this invention.

The brominated polyaromatic mixtures produced by the process of this invention are useful as flame retardants in ABS, i.e., acrylonitrile-butadiene-styrene, and HIPS, i.e., high impact polystyrene based formulations. The amount used to achieve the desired flame retarded effect, is generally from 12 weight percent to 25 weight percent, based upon the total weight of the formulation. It is preferred that the formulation also contain any of the well known flame retardant synergists which are commonly used with halogen containing flame retardants. Such synergists enhance the flame retardant qualities of the brominated polyaromatics in the mixture and thus enable the use of lesser amounts of the mixture to obtain the desired flame retardant effect. Examples of such synergists are: $Sb_2O_3$, $Sb_2O_4$, and $Sb_2O_5$, zinc oxide, zinc borate, various inorganic bismuth compounds and organic compounds, such as, tris-2-chloroethyl-phosphate and tris-2,3-dibromopropyl-phosphate. The most preferred synergist is $Sb_2O_3$.

The flame retardant synergist will generally be used in an amount, based upon the total weight of the ABS based formulation, which is within the range of from 2 weight percent to 6 weight percent. When a flame retardant synergist is used, the amount of brominated polyaromatic mixture used is preferably within the range of from 10 weight percent to 20 weight percent.

The ABS resin can be any of those which are denominated by the art as high impact, medium impact, low impact or heat resistant. The ABS resin can be comprised of any suitable proportion of acrylonitrile, rubber or styrene. The resin can also be any of those produced by the well known emulsion, suspension or batch processes. Even further, the resin may have units other than acrylonitrile, butadiene and styrene. For example, methylmethacrylate can be copolymerized therewith. Also, other polymers may be used to modify the ABS resin, such other polymers including modified styrene resins, such as rubber modified polystyrenes, and the styrene containing copolymers, such as the styrene-acrylonitrile copolymers, styrene-butadiene copolymers, styrene-acrylonitrile-$\alpha$-alkyl styrene copolymers, poly-$\alpha$-methyl styrene, copolymers of ethylvinylbenzene and divinylbenzene, and the like. The preferred resin is unmodified acrylonitrile-butadiene-styrene. For a further discussion of suitable ABS resins, see Kirk-Othmer Encyclopedia of Chemical Technology, 3rd edition, John Wiley & Sons, Vol. 1, pages 442-456, and Encyclopedia of Polymer Science and Technology, John Wiley & Sons, Vol. 1, pages 436-444.

The ABS resin substituent used in the formulation will comprise from 40 to 70 weight percent of the formulation and preferably from 50 to 60 weight percent.

The substituents of the ABS based formulation can be blended one with the other in any order and by way of any conventional technique. A Banbury mixer or twin screw extruder can be used.

The ABS based formulation can also contain conventional additives, for example, plasticizers, pigments, antioxidants, fillers, e.g. talc or glass, UV stabilizers, and processing aids.

Conventional article forming techniques can be used to form articles from the above described ABS based formulations. For example injection molding, compression molding, and extrusion molding are all suitable.

The following Examples illustrate some of the features of the inventions hereinabove disclosed and are not to be taken as limiting such inventions.

EXAMPLES

The following equipment was used in Examples I-V.

A 500 mL, 5-necked reaction flask was fitted with a Friedrich's condenser modified for use as a dry-ice/IPA cold-finger condenser. The reactor overhead led from the condenser exit to an oil bubbler charged with inert fluorocarbon oil, a safety trap and a tared caustic trap. The dip-leg to the caustic trap was positioned just below the liquid surface; the trap itself was placed on a balance to measure HBr evolution quantitatively with reaction time. Alternatively, the trap could be stirred magnetically if HBr weight was not a concern. A nitrogen line was tied into the overhead, downstream from the condenser. A 3-way Teflon stopcock allowed a $N_2$ purge to maintain positive pressure when HBr flow became weak, thus preventing caustic suck-back. To clear most of the residual HBr from the system, the nitrogen purge could be transferred to the 3-way stopcock on the side-arm of the empty bromine addition funnel. With the stopcock open, the addition funnel, reactor and condenser vapor spaces could be flushed at a controlled rate. The addition funnel itself was fitted with a 2 mm, metered Teflon stopcock. A thermocouple-thermowell was placed in the fourth reactor neck; the fifth neck was used for catalyst addition.

EXAMPLE I

The reactor was charged with 54.6 g, (0.30 mole) diphenylethane, hereinafter DPE, which was dissolved in 90 mL methylene bromide. Ferric bromide (2.7 g, 0.009 mole) was added. Bromine (287.6 g, 1.80 moles) was added over 1.0 hours at 20-40°C. After the bromine addition was complete, the mixture was heated to 50° to 60° C for 1.0 hour, yielding a thick slurry. Water (200 mL) was added to the reaction mass and the resulting slurry was heated to flash off the methylene bromide solvent.

The product solids rapidly formed as a thick aqueous slurry. The thick, off-white slurry was cooled, filtered, washed with water and dried to a constant weight to give an off-white powder (95.5% yield). The bromo homolog distribution and average bromine number are given in Table 1.

EXAMPLE II

To obtain the product distribution indicated in Table 1, the general procedure and catalyst of Example I was utilized.

EXAMPLE III

The general procedure of Example I was followed except that the bromination catalyst was $ZrCl_4$. The product obtained was pink in color and had the bromo homolog distribution and average bromine number given in Table 1.

EXAMPLE IV

The general procedure of Example I was followed except that the bromination catalyst was a mixture of $FeBr_3$ and $CH_3NO_2$ in a 1:1 molar ratio. The product obtained was off-white in color and had the bromo homolog distribution and average bromine number given in Table 1.

EXAMPLE V

(Comparative Example)

This example is not of this invention, but is given for comparative purposes. The reactor was charged with 27.3 g, (0.15 mole) diphenylethane, hereinafter DPE, which was dissolved in 45 mL methylene bromide. To the reaction mass $AlCl_3$ catalyst (1.38 g, 0.01 mole) was added and bromine addition was begun. Bromine (143.8 g, 0.90 mole) was added over 1.0 hour and 10 minutes at 10-33°C. After the bromine addition was complete, the mixture was heated to 45° C for 2.0 hours, yielding a thick slurry. Water (100 mL) was added to the reaction mass and the resulting slurry was heated to flash off the methylene bromide solvent.

The product solids rapidly formed as a thick, aqueous slurry. The thick, dark brown slurry was cooled, filtered, washed with water and dried to a constant weight. The bromo homolog distribution and average bromine number are given in Table 1.

TABLE 1

| Constituent | Product from Example No. | | | | |
|---|---|---|---|---|---|
| | I GC area % | II GC area % | III GC area % | IV GC area % | V GC area % |
| DPE-$Br_4$ | 0 | --- | --- | --- | 0.5 |
| DPE-$Br_5$ | 10.6 | 10.2 | 7.8 | 11.4 | 8.6 |
| DPE-$Br_6$ | 82.5 | 79.7 | 84.4 | 83.9 | 65.1 |
| DPE-$Br_7$ | 6.9 | 9.7 | 7.4 | 4.6 | 13.2 |
| DPE-$Br_8$ | --- | 0.4 | 0.2 | 0.1 | 1.8 |
| DPE-$Br_9$ | --- | --- | --- | --- | --- |
| DPE-$Br_{10}$ | --- | --- | --- | --- | --- |
| Light Ends | N.D. | N.D. | 0.3 | <0.1 | 10.8 |
| Melting Point Range (° C) | 173-195 | 175-196 | 168-197 | 175-194 | 105-174 |
| Average Br No.[1] | 6.0 | 6.0 | 6.0 | 5.9 | 6.1 |

[1] Average Br number for Examples obtained by normalizing GC area percent excluding Light Ends.

EXAMPLE VI

ABS-based formulations were prepared using a Brabender mixer. The formulations contained 4 weight percent $Sb_2O_3$, 58.75 weight percent Magnum® PG-914, an ABS resin sold by The Dow Chemical Company; 15 weight percent Tyrin®, a chlorinated polyethylene resin from the Dow Chemical Company; 3 weight percent $TiO_2$; 1 weight percent Tinuvin® 326, sold by Ciba-Geigy Corporation; 0.25 weight percent Tinuvin® 770 (Ciba-Geigy Corporation); and 18 weight percent of the flame retardant indicated. Each formulation was compression molded at a temperature of 177°C and at a molding pressure of 1400-1800 gram-meter torque to form test specimens which are identified in Table 2 in accordance with which formulation was used to produce which specimen.

TABLE 2

| Test Plaque | Formulation Composition | UL-94 Rating | ASTM D 256 Izod Impact 1/8" FT.lb/in. notch | UV-Stability $\Delta E_{48}$ |
|---|---|---|---|---|
| 1 | Example III mixture | V-0 | 3.7 | 15.5 |
| 2 | Example IV mixture | V-0 | 3.6 | 13.6 |
| 3 | Saytex® RB-100[1] | V-0 | 2.2 | 17.5 |
| 4 | GLC[2] FF-680 | V-0 | 3.8 | 12.1 |
| 5 | Saytex® S-111[3] | V-0 | 3.9 | 26.7 |

[1]Saytex® RB-100 - tetrabromobisphenol-A of Ethyl Corporation
[2]FF-680 - bis(tribromophenoxy)ethane of Great Lakes Corporation
[3]Saytex® S-111 - Octabromodiphenyl oxide of Ethyl Corporation

As can be seen from Table 2, a brominated diphenyl ethane mixture of this invention (Test Plaques 1 & 2) gave a UL-94 V-O rating with little adverse affect on the specimen's Izod Impact strength.

**Claims**

**1.** A process for preparing a mixture of brominated non-condensed ring polyaromatics having an average of about 6 bromine atoms per molecule and a reduced amount of light end impurities, which process comprises:

(a) adding bromine to a reactor initially containing a reaction mass comprising a solvent, a catalytic amount of a bromination catalyst, and a non-condensed ring polyaromatic of the formula

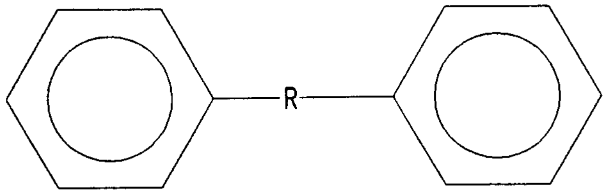

wherein R is an alkylene group of 1 to 10 carbon atoms, an oxygen atoms, a sulfur atom, an oxyalkylene group of up to 6 carbon atoms, an oxyalkyleneoxy group of up to 6 carbon atoms or a carbon single bond;

(b) maintaining the reaction mass during the bromine addition at a temperature of from 15° to 40°C;

(c) terminating the addition of bromine when sufficient bromine has been added to obtain the mixture of polyaromatics having an average of about 6 bromine atoms per molecule of polyaromatic; and

(d) after at least substantially all of the bromine has reacted, recovering the brominated non-condensed ring polyaromatic mixture from the reaction mass, the recovered mixture containing more than about 55 %, based on gas chromatographic area percent, of a brominated polyaromatic having about 6 bromine atoms per molecule.

**2.** The process of Claim 1 wherein the non-condensed ring polyaromatic is diphenyl oxide or 1,2-diphenylethane.

**3.** The process of Claim 1 or 2 wherein there are from 200 mLs to 400 mLs of solvent per mole of non-condensed ring polyaromatic and the solvent is methylene bromine, methylene chloride or a mixture thereof.

**4.** The process of any of the preceding claims wherein the bromination catalyst is an iron halide catalyst.

**5.** The process of Claim 4 wherein the bromination catalyst is, at least initially, ferric bromide, ferric chloride or a mixture of $FeBr_3$ and $CH_3NO_2$.

**6.** The process of Claim 4 wherein the amount of iron halide catalyst is within the range of from 2 to 5 wt.% based on the total weight of the non-condensed ring polyaromatic.

**7.** The process of any of the preceding claims wherein the amount of bromine added to the reaction mass is from 5.8 to 6.2 moles of bromine per mole of non-condensed ring polyaromatic.

**8.** The process of any of the preceding claims wherein the non-condensed ring polyaromatic is 1,2-diphenylethane and wherein the recovered mixture, when subjected to gas chromatography (GC), will yield a gas chromatogram is which there is indicated from 5 to 12 GC area percent pentabromodiphenylethane; from 75 to 90 GC area percent hexabromodiphenylethane; from 3 to 10 GC area percent heptabromodiphenylethane; and from 0 to 1.0 GC area percent octabromodiphenylethane.

**9.** A flame retardant product comprising a mixture of brominated non-condensed ring polyaromatics having an average of about 6 bromine atoms per molecule and a melting point range of from 160 to 200° C.

**10.** A flame retardant product comprising a mixture of brominated diphenylethane, wherein the mixture contains from 5 to 12 GC area percent pentabromodiphenylethane; from 75 to 90 GC area percent hexabromodiphenylethane; from 3 to 10 GC area percent heptabromodiphenylethane; and from 0 to 1 GC area percent octabromodiphenylethane.

European Patent Office

# EUROPEAN SEARCH REPORT

Application Number

EP 92 10 2060

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | US-A-5 041 687 (BONNIE G. MCKINNIE ET AL.) * column 1, line 1 - column 3, line 60; claims 1-3,5-14; example VI; table I * --- | 1-7,9-10 | C07B39/00 C07C17/10 C07C25/18 C07C43/29 C08K5/03 |
| P,A | EP-A-0 445 595 (ETHYL CORPORATION) * claims; examples * --- | 1-10 | |
| A | EP-A-0 299 270 (ETHYL CORPORATION) * claims; examples * ----- | 1-10 | |
| | | | TECHNICAL FIELDS SEARCHED (Int. Cl.5) |
| | | | C07C C07B |

The present search report has been drawn up for all claims

| Place of search | Date of completion of the search | Examiner |
|---|---|---|
| THE HAGUE | 01 JULY 1992 | ZERVAS B. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)